# EUROPEAN PATENT APPLICATION

(11) **EP 3 456 259 A1**
(43) Date of publication of application: **20.03.2019**
(21) Application number: 18193465.4
(22) Date of filing: 10.09.2018
(51) Int. Cl.: A61B 5/08, H04R 25/00, G02C 11/00

(54) **METHOD, APPARATUS, AND COMPUTER PROGRAM FOR ADJUSTING A HEARING AID DEVICE**

(30) Priority: 15.09.2017 EP 17191272
(71) Applicant: Oticon A/S, 2765 Smørum (DK)
(72) Inventor: WENDT, Dorothea, DK-2765 Smørum (DK); CLEVELAND NIELSEN, Annette, DK-2765 Smørum (DK); GRAVERSEN, Carina, DK-2765 Smørum (DK); GRIFUL, Sergi Rotger, DK-2765 Smørum (DK)
(74) Representative: William Demant

(57) **Abstract**

The present disclosure relates to a concept for adjusting a hearing aid device. A sound pattern is provided to a person wearing the hearing aid device configured according to current hearing aid settings. The person's pupils are evaluated to obtain pupillary information in response to the sound pattern perceived via the hearing aid device. The current hearing aid settings are adjusted based on the pupillary information.

## Description

### TECHNICAL FIELD

The present disclosure generally relates to hearing aid devices and, more particularly, to adjusting or calibrating hearing aid devices in accordance with user specific needs.

### BACKGROUND

Modern hearing aids require configuration to match specific hearing loss, physical features, and lifestyle of the wearer. This process is called "fitting" and is typically performed by audiologists who treat persons with hearing loss and proactively prevents related damage. The fitting and fine-tuning of hearing aids today is not evidence based, as audiometric tests used today in essence are based on self-reported data. This is also true for the outcome data on hearing aid satisfaction as they are based on self-reported questionnaires. Self-reported data can be prone to several biases, such as recall bias, prestige bias, and misclassification bias, which can all cloud the outcome measure's reliability and validity and thus can hinder evidence based clinical decision support. Moreover, traditional audiometric tests used in the clinic today can have limitations as tone detection in the brain does not solely reflect how good speech is perceived, and how speech perception requires listening effort. Fitting to the audiometric tests does also not take into account individual cognitive efforts and problems in processing the sounds encountered.

Traditionally, almost all hearing healthcare services are provided through physical meetings between hearing aid users and audiologists, which is time-, transportation-, and resource-consuming for all parties. Moreover, people tend to forget 40-80% of the information given during health visits. "eHealth" is a term for healthcare practice supported by electronic processes and communication. Such eHealth solutions aim at empowering people in need of healthcare to self-manage with support from their audiologist or a cloud computing service through eHealth solutions. Furthermore, eHealth solutions encourage healthcare self-management by making information easily accessible at all times and in the user's own pace at home.

Fitting and fine-tuning of hearing aids today is not individualized through pre-fitting/fine-tuning information from users, nor have audiologists access to evidence based and individualized objective outcome measures data pre-, and post-fitting, which could enable clinical decision support for individualized preplanning of the fitting process and on-demand support and problem solving post-fitting, the latter especially relevant in the first two to four weeks' acclimatization period.

The users have very little knowledge of the user journey, individual expectations for hearing aids and important listening situations, listening preferences etc., which could otherwise empower them in their rehabilitation process, and reduce time in the clinic. This lack of information, learning, and communication sharing between users and audiologists throughout the user journey give rise to some problems: the audiologists do not know anything about the user before they enter the clinic for a fitting session and thus have no data supporting a planning and preparation of an individualized fitting process. Today user information and data on listening preferences, important listening situations etc., are obtained through the short face-to-face visit in the clinic. This information could be provided to the audiologist before fitting enabling individualized fitting. All these could very well contribute to the 24% non-use of hearing aids (8% of hearing aid users do not at all use their hearing aids and another 16% use them less than 1 hour a day), a high return for credit, and way too many revisits in the clinic all of which is counterproductive in clinic cost-efficiency and user satisfaction.

When the user moves out of the controlled settings in the clinic and out in the user real-life listening situations, many users experience a mismatch between the clinical settings listening situations testing and real-life experiences listening situations creating dissatisfaction with hearing aids and possible non-use.

Thus, there is a need for improvements in the technical field of hearing aid fitting/adjustment.

### SUMMARY

This need is satisfied by methods, apparatuses, and computer programs according to the independent claims. Further advantageous examples are addressed by the dependent claims.

According to a first aspect, the present disclosure provides a method for adjusting a hearing aid device/instrument. Thereby the hearing aid device can be understood as an electroacoustic device that transforms environmental sound (including spoken words) to make it more intelligible or comfortable for a user, according to audiometric and cognitive rules. The proposed method includes providing a sound pattern to a person wearing the hearing aid device that is configured according to current hearing aid settings. The method further includes evaluating the person's pupils to obtain pupillary (or pupillometric) information in response to the sound pattern perceived via the hearing aid device, and adjusting the current hearing aid settings based on the pupillary information.

The evaluation of the person's pupils includes recording of the pupil size, post-processing/cleaning of the recorded data and analysis of the recorded pupil curve to estimate a value of the listening effort, i.e. the cognitive load.

The skilled person having benefit from the present disclosure will appreciate that the hearing aid settings can comprise audio filter settings for environmental sound transformation. That is to say, a first hearing aid setting leads to a first (frequency) transfer function of the hearing aid device while a different second hearing aid setting leads to a different second (frequency) transfer function of the hearing aid device. The proposed concept can be used to objectively adjust the hearing aid settings and thus the transfer characteristic of the hearing aid device so as to minimize the user's cognitive load for listening to the sound pattem(s) and for avoiding subjective assessment of the adjustment of the hearing aid settings and thus the transfer characteristic.

In some examples, the sound pattern(s) can be predefined by the manufacture of the hearing aid device, which means that the sound patterns are not arbitrary or random sound patterns but selected and/or pre-recorded sound patterns. This can increase the reliability and/or objectiveness of the proposed pupillometric hearing aid fitting concept.

In some examples, providing the sound pattern can include playing (predefined) speech samples to the person wearing the hearing aid device. Optionally, a predefined background sound corresponding to a given (ambient) sound environment may be played in the hearing aid device for performing a baseline measurement of the pupil dilation of the person wearing the hearing aid device. The predefined speech samples may be played together with the predefined background sound. The person's pupil dilation will then be tracked/recorded by using an eye tracking camera while listening to the speech samples together with the background sound. The recorded pupil dilation will then be post-processed, including for example eye-blink removal and normalization of the measured pupillary information, e.g. the pupil size and/or the pupil dilation, data interpolation and analyzed to obtain a measure for the individual's listening effort. Particularly, difficult and/or individually important hearing situations can be simulated by the predefined speech samples and/or the predefined background sound/noise.

In some examples, providing the sound pattern comprises transmitting data indicative of the (predefined) sound pattern and/or background sound from a remote device to a mobile device associated with the person wearing the hearing aid device. In this way, remote fitting can be supported. For example, this mobile device can be a laptop computer, a smartphone, or any other device configured to play back the predefined sound pattern and/or background sound/noise to the person wearing the hearing aid device. In some examples, the data indicative of the (predefined) sound pattern and/or background sound may even be directly transmitted to the hearing aid device itself. The skilled person will appreciate that the hearing aid device would then have to comprise adequate transceiver circuitry to receive and play back the data. The remote device could for instance be a remote server, for example a cloud server storing one or more (predefined) sound patterns.

In some examples, the sound pattern could have been generated and/or stored by the person herself via the mobile device, such as a smartphone, tablet or a video camera. The sound pattern can then also be played back via the user's mobile device, for example.

In some examples, the person's pupils can be evaluated automatically, for instance by using an automatic eye tracking device and/or a pupilometer for measuring the person's pupil dilation as an indicator of their cognitive load. The eye tracking device may comprise an infrared camera for focusing on one or both eyes and record their movement and/or change in the pupil dilation while the user listens to the sound pattern(s). The eye-tracking device can use the center of the pupil and infrared / near-infrared non-collimated light to create corneal reflections (CR), for example. An additional or alternative automated pupilometer can be a portable, handheld infrared device that can provide a reliable and objective measurement of pupillary size, and reactivity through measurement of the pupil dilation.

In order to perform the measurement of the pupil dilation via an infrared camera the light condition around the person's eyes has to be known. That may be accomplished by measuring the light intensity around the eye via the same infrared camera or by placing the person under in a dark room. The eye tracking device or pupilometer can comprise pupillometric goggles or alike or other device. The goggles or alike can comprise one or more cameras and one or more IR radiation sources, for example.

In some examples, adjusting the current hearing aid settings comprises varying the current hearing aid settings to decrease a measured pupil dilation in response to the (predefined) sound pattern and/or background sound/noise. That is to say, the hearing aid is adjusted in a way that the peak of the pupil dilation decreases indicating that the person is spending effort for processing the sound stimulus. For remote fitting, the hearing aid settings can be adjusted or selected by a remote processor, for example.

In some examples, adjusting the current hearing aid settings comprises selecting the current hearing aid settings from a plurality of different predetermined hearing aid settings and selecting the hearing aid settings causing the least pupil dilation in response to the (predefined) sound pattern.

While the above method(s) can also be performed at least partially manually, some examples also propose fully automated and potentially remote implementations. For this purpose, the method can further comprise transmitting the measured pupil dilation to a processor, where the processor is configured to post-process the pupil dilation for eye blink removable, artifact removable, data interpolation and normalization of the pupil dilation. The processor provides then adjusted hearing aid settings based on the post-processed pupil dilation. The processor can be implemented in the hearing aid device itself or it can be located remotely from the hearing aid device, for example of a remote (cloud) server or a mobile device (e.g., a smartphone) associated with the hearing impaired user. In the latter case, the providing of the adjusted hearing aid settings can include transmitting the adjusted hearing aid settings from the remote processor to the hearing aid device. Thus, also cloud services can be employed for fitting of hearing aids.

According to a further aspect, the present disclosure also provides a computer program for performing implementations of the aforementioned method(s), when the computer program is executed on a programmable hardware device, such as a computer, a Field Programmable Gate Array (FPGA), or an Application Specific Integrated Circuit (ASIC), for example. According to yet a further aspect, the present disclosure also provides an apparatus for adjusting a hearing aid device. The apparatus comprises receiver circuitry which is configured to receive pupillary information of a person wearing the hearing aid device configured according to current hearing aid settings. The apparatus further comprises processor circuitry which is configured to generate adjusted hearing aid settings based on the pupillary information. The skilled person having benefit from the present disclosure will appreciate that such an apparatus can be integrated in the hearing aid device itself or be located remote from the hearing aid device. In some examples, it can be a remote computer or a smartphone which can be used for fitting purposes.

According to yet a further aspect, the present disclosure also provides a system for adjusting a hearing aid device. The system comprises means for providing a (predefined) sound pattern to a person wearing the hearing aid device configured according to current hearing aid settings, means for evaluating the person's pupils to obtain pupillary information in response to the (predefined) sound pattern perceived via the hearing aid device, and means for adjusting the current hearing aid settings based on the pupillary information.

In some examples, the system may comprise a mobile device (e.g. a smartphone) including an application software configured to provide the (predefined) sound pattern to the hearing aid device or a headphone or to the means for evaluating the person's pupils. In some examples, the pupillary information may be transmitted to either the hearing aid device or to a remote device via a mobile device or server/cloud. In case the pupillary information is transmitted to the hearing aid device, the hearing aid device can be configured to select appropriate hearing aid settings based on a detected sound environment (via a microphone within the hearing aid device) based on the pupillary information and the predefined background sound sent by cloud/server.

In the situation where the remote device receives the pupillary information, an audiologist can perform remote fine tuning/remote fitting of the hearing aid settings via a communication link established between the hearing aid device and the remote device. The communication link may for instance be a Bluetooth link, or a mobile telephone communication link, or an internet communication link or a combination thereof. Examples of the present disclosure propose evidence based and individualized fitting and/or fine-tuning beyond an audiogram by pupillometry and eHealth in the clinic or at home. Pupillometry can be used as a diagnostic tool measuring the pupil dilation of a person and interpret the data of the measured pupil dilation for determine a hearing aid setting influencing a hearing profile of the hearing aid device. A baseline measurement of the pupil dilation of the person may be performed just before doing the actual measure of the pupil dilation during playing the sound pattem(s).

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
- Figs. 1a to 1d: show different setups for hearing aid fitting based on pupillometry;
- Fig. 1e: illustrates a flowchart of a method for adjusting a hearing aid device;
- Figs. 2a and 2b: show a message sequence chart according to different examples of implementations;
- Fig. 3: shows an eHealth system for hearing aid fitting based on pupillometry;
- Fig. 4: shows a normalized pupil dilation;
- Fig. 5: illustrates a flowchart of a method for emulating hearing loss; and
- Fig. 6: shows an embodiment of a hearing aid system according to the present disclosure comprising left and right hearing device and a number of sensors mounted on a spectacle frame.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the apparatus and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

A hearing aid device may include a hearing aid that is adapted to improve or augment the hearing capability of a user by receiving an acoustic signal from a user's surroundings, generating a corresponding audio signal, possibly modifying the audio signal and providing the possibly modified audio signal as an audible signal to at least one of the user's ears. The "hearing aid device" may further refer to a device such as an earphone or a headset adapted to receive an audio signal electronically, possibly modifying the audio signal and providing the possibly modified audio signals as an audible signal to at least one of the user's ears. Such audible signals may be provided in the form of an acoustic signal radiated into the user's outer ear, or an acoustic signal transferred as mechanical vibrations to the user's inner ears through bone structure of the user's head and/or through parts of middle ear of the user or electric signals transferred directly or indirectly to cochlear nerve and/or to auditory cortex of the user.

The hearing aid device is adapted to be worn in any known way. This may include i) arranging a unit of the hearing aid device behind the ear with a tube leading airborne acoustic signals or with a receiver/ loudspeaker arranged close to or in the ear canal such as in a Behind-the-Ear type hearing aid or a Receiver-in-the Ear type hearing aid, and/ or ii) arranging the hearing aid device entirely or partly in the pinna and/ or in the ear canal of the user such as in an In-the-Ear type hearing aid or In-the-Canal/ Completely-in-Canal type hearing aid, or iii) arranging a unit of the hearing aid device attached to a fixture implanted into the skull bone such as in Bone Anchored Hearing Aid or Cochlear Implant, or iv) arranging a unit of the hearing aid device as an entirely or partly implanted unit such as in Bone Anchored Hearing Aid or Cochlear Implant.

A hearing aid device may be part of a "hearing system", which refers to a system comprising one or two hearing aid devices, disclosed in present description, and a "binaural hearing system" refers to a system comprising two hearing aid devices where the devices are adapted to cooperatively provide audible signals to both of the user's ears. The hearing system or binaural hearing system may further include auxiliary device(s) that communicates with at least one hearing aid device, the auxiliary device affecting the operation of the hearing aid devices and/or benefitting from the functioning of the hearing aid devices. A wired or wireless communication link between the at least one hearing aid device and the auxiliary device is established that allows for exchanging information (e.g. control and status signals, possibly audio signals) between the at least one hearing aid device and the auxiliary device. Such auxiliary devices may include at least one of remote controls, remote microphones, audio gateway devices, mobile phones, public-address systems, car audio systems or music players or a combination thereof. The audio gateway is adapted to receive a multitude of audio signals such as from an entertainment device like a TV or a music player, a telephone apparatus like a mobile telephone or a computer, a PC. The audio gateway is further adapted to select and/or combine an appropriate one of the received audio signals (or combination of signals) for transmission to the at least one hearing aid device. The remote control is adapted to control functionality and operation of the at least one hearing aid devices. The function of the remote control may be implemented in a Smartphone or other electronic device, the Smartphone/ electronic device possibly running an application that controls functionality of the at least one hearing aid device.

In general, a hearing aid device includes i) an input unit such as a microphone for receiving an acoustic signal from a user's surroundings and providing a corresponding input audio signal, and/or ii) a receiving unit for electronically receiving an input audio signal. The hearing aid device further includes a signal processing unit for processing the input audio signal and an output unit for providing an audible signal to the user in dependence on the processed audio signal.

The input unit may include multiple input microphones, e.g. for providing direction dependent audio signal processing. Such directional microphone system is adapted to enhance a target acoustic source among a multitude of acoustic sources in the user's environment. In one aspect, the directional system is adapted to detect (such as adaptively detect) from which direction a particular part of the microphone signal originates. This may be achieved by using conventionally known methods. The signal processing unit may include amplifier that is adapted to apply a frequency dependent gain to the input audio signal. The signal processing unit may further be adapted to provide other relevant functionality such as compression, noise reduction, etc. The output unit may include an output transducer such as a loudspeaker/ receiver for providing an air-borne acoustic signal transcutaneously or percutaneously to the skull bone or a vibrator for providing a structure-borne or liquid-borne acoustic signal. In some hearing aid devices, the output unit may include one or more output electrodes for providing the electric signals such as in a Cochlear Implant.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

Accordingly, the scope should be judged in terms of the claims that follow.

Various examples will now be described more fully with reference to the accompanying drawings in which some examples are illustrated. In the figures, the thicknesses of lines, layers and/or regions may be exaggerated for clarity.

Accordingly, while further examples are capable of various modifications and alternative forms, some particular examples thereof are shown in the figures and will subsequently be described in detail. However, this detailed description does not limit further examples to the particular forms described. Further examples may cover all modifications, equivalents, and alternatives falling within the scope of the disclosure. Like numbers refer to like or similar elements throughout the description of the figures, which may be implemented identically or in modified form when compared to one another while providing for the same or a similar functionality.

It will be understood that when an element is referred to as being "connected" or "coupled" to another element, the elements may be directly connected or coupled or via one or more intervening elements. If two elements A and B are combined using an "or", this is to be understood to disclose all possible combinations, i.e. only A, only B as well as A and B. An alternative wording for the same combinations is "at least one of A and B". The same applies for combinations of more than 2 Elements.

The terminology used herein for the purpose of describing particular examples is not intended to be limiting for further examples. Whenever a singular form such as "a," "an" and "the" is used and using only a single element is neither explicitly or implicitly defined as being mandatory, further examples may also use plural elements to implement the same functionality. Likewise, when a functionality is subsequently described as being implemented using multiple elements, further examples may implement the same functionality using a single element or processing entity. It will be further understood that the terms "comprises," "comprising," "includes" and/or "including," when used, specify the presence of the stated features, integers, steps, operations, processes, acts, elements and/or components, but do not preclude the presence or addition of one or more other features, integers, steps, operations, processes, acts, elements, components and/or any group thereof. Unless otherwise defined, all terms (including technical and scientific terms) are used herein in their ordinary meaning of the art to which the examples belong.

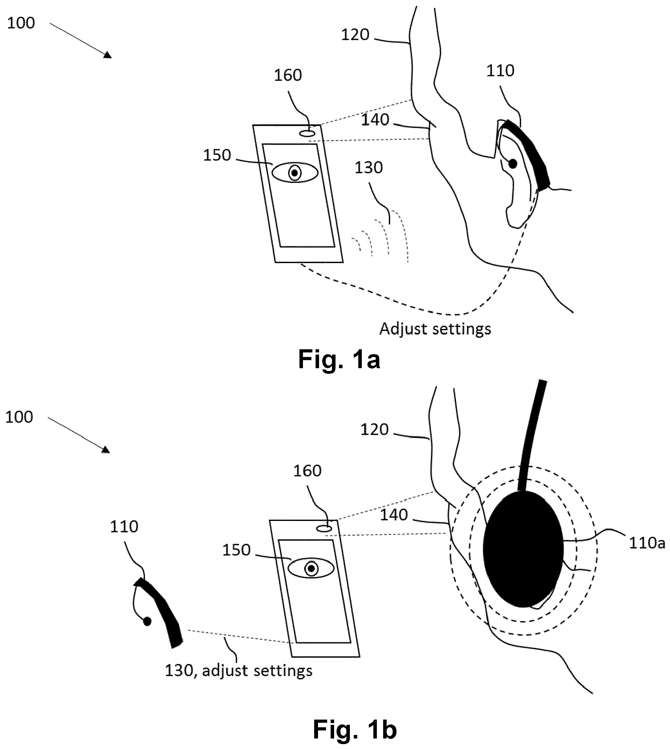

Figs 1a to 1d show different setups for hearing aid fitting based on pupillometry. Fig. 1a shows a basic setup 100 which can be used to adjust the settings of an electroacoustic hearing aid device 110 worn by a hearing impaired person 120.

In Fig. 1a, the user 120 or the person under test 120 is wearing the hearing aid device 110 and looking into a pupilometer 160 or an eye tracking camera 160, which in this specific example is part of a smartphone including a graphical display for displaying the pupillary information 150, i.e. the pupil size or the pupil dilation. The hearing aid device 110 can then be modified/adjusted based on the measured pupillary information, i.e. the pupil size and/or pupil dilation.

In fig. 1b, the user 120 is wearing a headphone 110a receiving the sound pattern from for example a smartphone., and the hearing aid device 110 is then configured to receive the modified/adjusted hearing aid settings via a digital audio playback device, such as a smartphone.

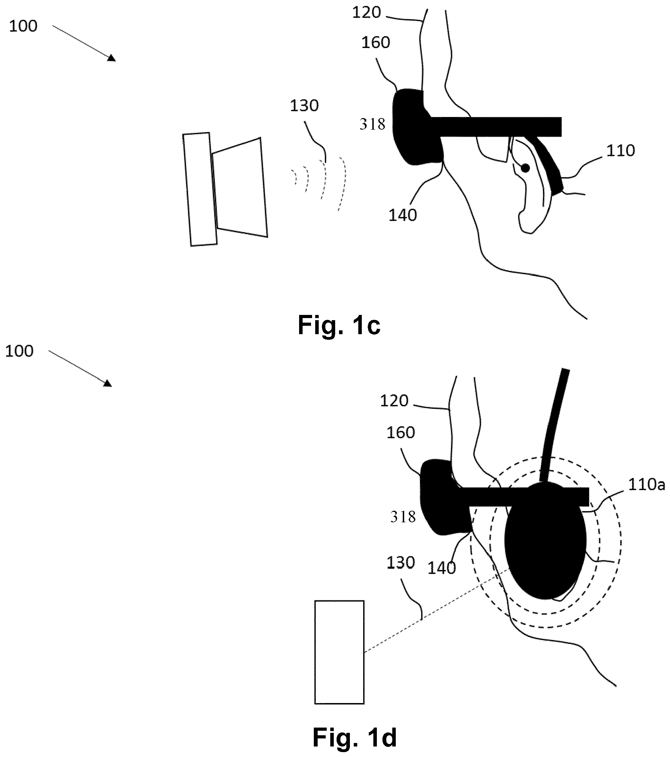

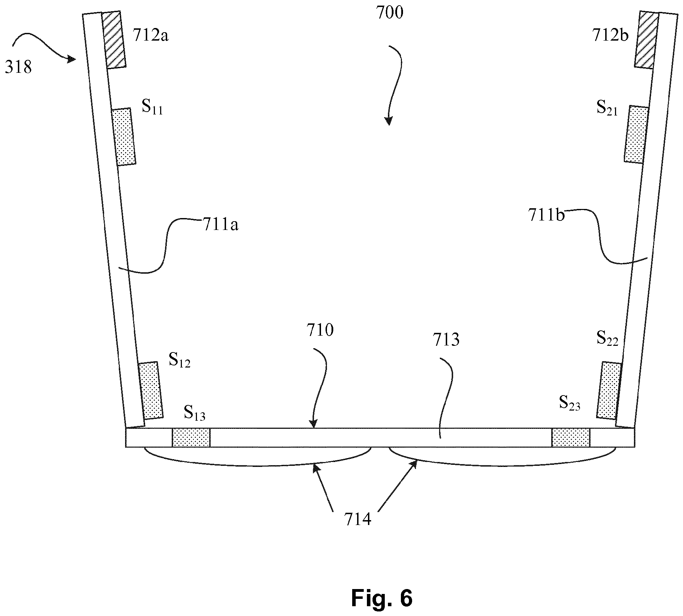

Fig. 1c illustrates an example where the user 120 is wearing the hearing aid device 110 and a goggle 318, also illustrated in fig 6, including the pupilometer or the eye tracking sensor 160. In this example the sound pattern comes from an external speaker. In another example the speaker may be built into the goggle or the hearing aid device 110 may comprise a signal generator or may be configured to receive the sound pattern 130 from a digital audio playback device.

Fig. 1d illustrates an example where the user 120 is wearing the headphone 110a and a goggle 318, also illustrated in fig 6, including the pupilometer 160 or the eye tracking sensor 160. In this example the headphone 110a receives the sound pattern 130 from a digital audio playback device.

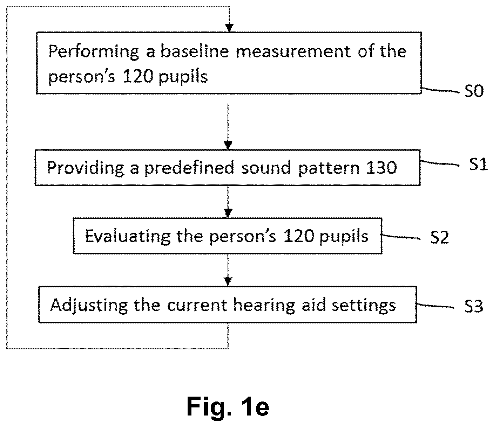

The hearing aid device 110 is configured according to current hearing aid settings influencing a hearing profile of the hearing aid device 110. As shown in the flowchart of Fig. 1e, a background sound is provided to the person and a baseline measurement is performed of the person's pupil in response to the background sound (see act S0). Then, a sound patterns 130 can be provided to the person 120 wearing the hearing aid device 110 (see act S1). Then, the person's 120 pupils 140 can be evaluated to obtain pupillary (or pupillometric) information 150 in response to the sound pattern 130 mixed with the background sound perceived via the hearing aid device 110 which is configured according to the current hearing aid settings (see act S2). Based on the pupillary information 150, the current hearing aid settings can be modified/adjusted (see act S3). The steps act S0 to S3 may be repeated with different sound patterns 130 and a further adjustment of the hearing aid settings may be necessary.

A basic underlying idea of the present disclosure hence is to use pupillary information 150 as an objective indicator of the hearing impaired person's 120 cognitive load for identifying the content of the sound pattern 130 perceived with the assistance of the hearing aid device 110. Here, the sound patterns 130 can be regarded as an audio stimulus and the measured pupillary information 150 comprises the person's 120 pupillary response due to the task of understanding/processing the audio stimulus. An objective is to adjust the hearing aid settings such that the pupillary information 150 indicates a possibly low cognitive load for identifying the sound pattern 130.

In some examples, the sound patterns 130 can comprise one or more speech patterns and/or an ambient sound environment. While the sound patterns 130 need not necessarily be predetermined or predefined, adjustment results may get better when using predefined sound or speech patterns. Such predefined sound or speech patterns may have been recorded and stored earlier, for example in a given sound environment, and may be played back to the hearing impaired person 120 later and potentially multiple times for fitting the hearing aid device 110. For the purpose of playback, a variety of electroacoustic transducers in proximity to the hearing impaired person 120 may be used, such as loudspeakers or headphones, for example. Such electroacoustic transducers may be coupled to an analog or digital audio playback device, such as a desktop computer, a laptop computer, a smartphone, or any other suitable device.

The pupillary information 150, such as pupil dilation, may be determined by measuring the dilation or constriction of the pupil(s) with an eye tracking device or a dedicated pupilometer 160. Thus, the person's pupils 140 can be evaluated by automatically measuring a pupil dilation in response to trying to identify the sound pattern 130. The eye tracking device or pupilometer 160 may be stationary (for example in a clinic) or portable and/or wearable. In some examples, the eye tracking device or pupilometer 160 may be integrated into a smartphone comprising a camera and loudspeakers for playing back the sound pattern 130. In other examples, the eye tracking device or pupilometer 160 may be integrated into a virtual reality headset comprising goggles and/or headphones for playing back the sound pattern 130 or other devices. In this way, the person 120 may be placed in an exactly predetermined and controllable audiovisual environment during the fitting procedure.

The measured pupillary information 150 may be transmitted to a signal processor for further processing. This signal processor may then generate adjusted hearing aid settings based on the measured pupillary information 150. In order to apply the adjusted hearing aid settings, the processor may be coupled to the hearing aid device 110 via either a wired or wireless communication link. To make the fitting procedure as comfortable and/or flexible as possible a wireless communication link is preferred. Examples of wireless technologies are Bluetooth or IEEE 802.11 (WLAN). In some examples, the processor for adjusting the hearing aid settings may be integrated in the hearing aid device 110 itself or be located remote from the hearing aid device. For instance, the processor for adjusting the hearing aid settings may be integrated in the same device as the eye tracker or pupilometer 160 or the processor may be integrated in a smartphone or a virtual reality headset. In other examples, the processor may be a remote processor, for example a remote server of a cloud service. Thus, some examples of the present disclosure may allow for remotely fitting the hearing aid device 110. Such examples will be detailed further below.

The hearing aid settings may be adjusted by varying currently applied hearing aid settings in order to decrease the measured pupil dilation in response to identifying the (predefined) sound pattern 130. That is to say, a (predefined) sound or speech pattern 130 may be played back to the hearing impaired person 120 wearing the hearing aid 110 with currently set first hearing aid settings. These first hearing aid settings lead to a first (frequency) transfer function of the hearing aid 110, leading to a first listening experience and a pupil dilatation giving an indicator of the cognitive load of the hearing impaired person 120. This first cognitive load translates into a first pupillary response while listening to a predefined sound or speech pattern 130. This first pupillary response is measured via eye tracking device or pupilometer 160. Then, second hearing aid settings may be applied to the hearing aid 110 leading to a second (frequency) transfer function of the hearing aid 110. Listening to the predefined sound or speech pattern 130 with the second hearing aid settings will lead to a second listening experience and cognitive load of the hearing impaired person 120 which then translates into a second pupillary reaction in response to the predefined sound or speech pattern 130. This second pupillary reaction may be measured via eye tracking device or pupilometer 160 and compared to the first pupillary reaction. After having applied a plurality of different hearing aid settings, those hearing aid settings leading to pupillary information 150 indicating the least cognitive load may be selected. Thus, adjusting the current hearing aid settings can comprise selecting the current hearing aid settings from a plurality of different predetermined hearing aid settings and selecting those hearing aid settings which have caused the least pupil dilation in response to the (pre-defined) sound pattern 130.

The pupil response, i.e. the pupil size or the pupil dilation, is measured in two (or more) different conditions, that means in different acoustic scenarios or with different hearing aid settings etc. The pupil response for the different conditions is then used as an indicator of the listening effort, i.e. the cognitive load, that was spend by the user in order to recognize the sound pattern, i.e. the sound or speech stimulus.

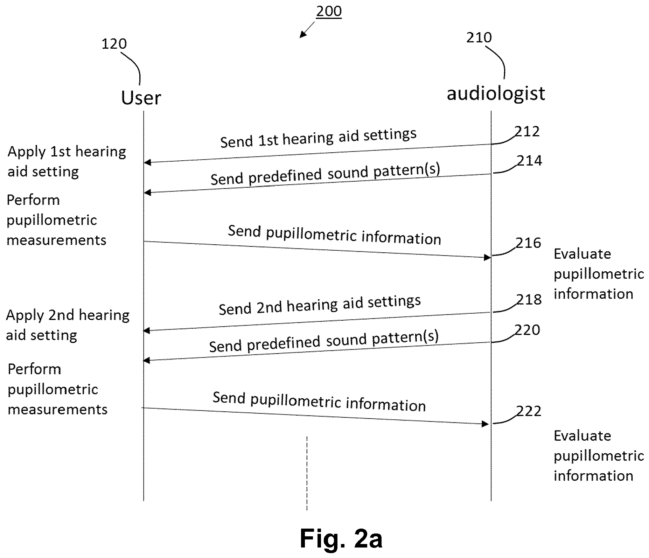

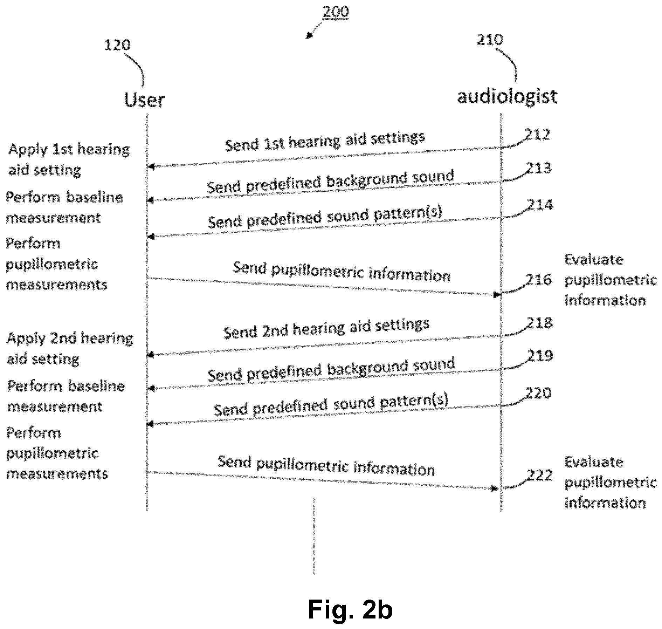

Figs. 2a and 2b illustrate different examples of implementations where the adjustment/fitting of the hearing aid device 110 may be done remotely or none-remotely. Remotely adjustment or fitting is defined as an adjustment or fitting of the hearing aid device where the sound pattern and the adjustment or fitting settings of the hearing aid device are communicated via a cloud based server and/or via a long distance communication network, such as telephone network, internet, WIFI etc.

The skilled person having benefit from the present disclosure will appreciate that the proposed concepts can lend itself to remote fitting concepts, where an audiologist can be at a different, remote location or does not even have to be involved at all. Fig. 2a schematically illustrates an example sequence chart 200 for remotely fitting the hearing aid device 110. As a first act, a hearing impaired user 120 receives a message 212 from a remote site 210 to apply first hearing aid settings. The message 212 can for example be received by a mobile device associated with the user 120, such as a user's smartphone or the hearing aid device itself. Message 212 can comprise instructions to apply first hearing aid settings stored at the user side, for example stored in the user's smartphone or hearing aid, or the message 202 itself could contain the first hearing aid settings. The remote site 210 could be a computer/server of a remote audiology lab controlled by an audiologist, for example. The remote site and the user 120 could be connected via internet, for example. For this purpose, an internet capable user device, such as a smartphone, computer, or a virtual reality headset, could be associated with the hearing impaired user 120. The user device can then adjust the current hearing aid settings in accordance with the first hearing aid settings. This can be done via a wired or wireless communication link between the user device and the hearing aid 110, for example.

Then, after having applied the first hearing aid settings to the user's hearing aid, the user device can be instructed via a message 214 to playback one or more predefined sound patterns. The playback could be done via a smartphone, a laptop or desktop computer, or the like. Message 214 could comprise instructions to playback one or more predefined sound patterns stored at the user side, or the message 202 itself could contain one or more predefined digital sound patterns.

Pupillometric measurements (e.g. pupil dilation) are then performed at the user side in order to measure the user's cognitive load for listening to and identifying the one or more predefined sound patterns with the first hearing aid settings. As mentioned above, the pupillometric measurements can be performed using an eye tracker or pupilometer 160, which can be integrated into a smartphone, a PC, a tablet, or a virtual reality headset worn by the user 120, for example. The pupillometric measurement may then be post-processed involving eye-blink removal and normalization of the measured pupillary information, e.g. the pupil size and/or the pupil dilation, data interpolation and analyzed to obtain a measure for the individual's listening effort. The pupillometric measurement results for the first hearing aid settings can then be send back to the remote site via message 216.

Next, the hearing impaired user 120 can receive a further message 218 from the remote site 210 to apply different second hearing aid settings. The current hearing aid settings can then be adjusted according to the second hearing aid settings. After having applied the second hearing aid settings, the user device can be instructed via a message 220 to playback the one or more predefined sound patterns. Pupillometric measurements are then performed again in order to measure the user's cognitive load for listening to and identifying the one or more predefined sound patterns with the second hearing aid settings. The pupillometric measurement results for the second hearing aid settings can then be send back to the remote site via message 222 after the post-processing of the measured pupillary information.

This procedure can be repeated multiple times for different potential hearing aid settings. After pupillometric measurement results for all potential hearing aid settings have been collected, the remote site 210 can evaluate the pupillometric measurement results and select the optimum hearing aid settings which have led to the pupillometric measurement results indicating the least cognitive load for listening to and identifying the one or more predefined sound patterns. These optimum hearing aid settings can then be applied.

Fig.2b schematically illustrates a similar example of a sequence chart 200 for remotely fitting the hearing aid device 110. In fig. 2b the user wearing either a hearing aid device 110 or a headphone 110a receives then a predefined background sound 213 which is then played in the hearing aid device 110 or in the headphone 110a. The baseline measurement of the pupil dilation, i.e. pupillary information, is provided based on the background sound. The user 120 receives then the sound patterns 214, and the pupillometric measurement is performed based on the received sound pattern in combination with the background sound. The measured pupillometric information is then transmitted back to the audiologist (210, 216). The baseline measurement is done just before each pupillometric measurement. The skilled person having benefit from the present disclosure will appreciate that the remote site of Figs. 2a and 2b could also be replaced by a smartphone or tablet or PC associated with the hearing impaired user. This smartphone or tablet or PC could establish a wireless connection with the user's hearing aid device and control the fitting procedure by applying different hearing aid settings, playing back the sound patterns, and performing the pupillometric measurements. In such examples, the smartphone or PC or tablet could execute a respective fitting app.

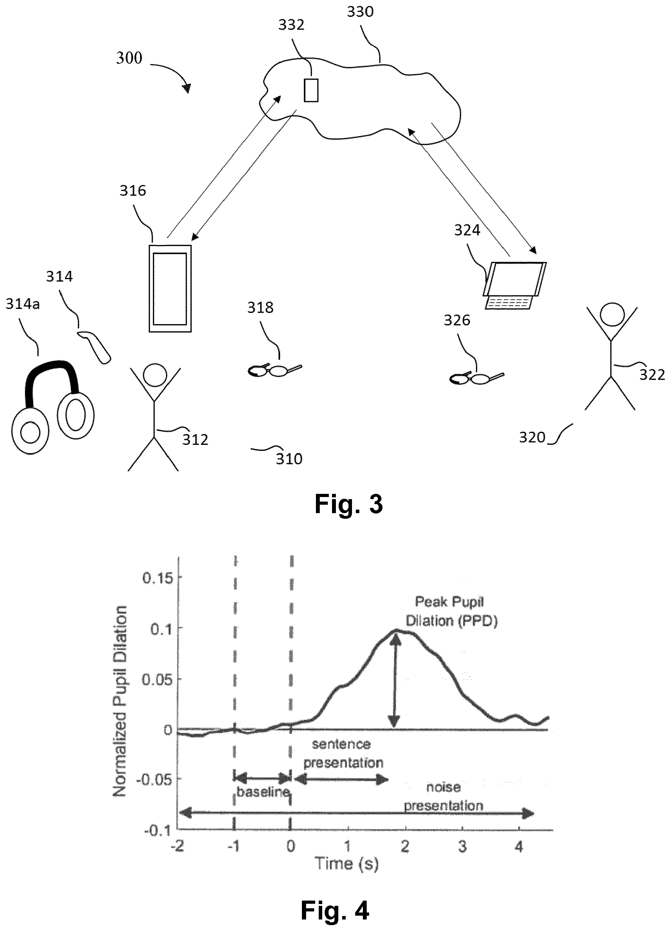

Today's problems with non- individualized hearing loss rehabilitation and lack of information and communication sharing could also be solved by using an eHealth ecosystem 300, as shown in Fig. 3, where information, communication and learning, i.e., eHealth flows between the user and their hearing aids to/from a cloud service and further to/from the audiologist.

The example eHealth ecosystem 300 of Fig. 3 comprises a hearing aid user domain 310, a hearing care professional domain 320, and a cloud service provider domain 330. Hearing aid user domain 310 includes a hearing aid user 312, a hearing aid device 314 or a headphone 314a, a portable user device 316 in form of a smartphone, PC, or tablet and a pupillometric measurement device comprising goggles 318 or PC or tablet or smartphone. Hearing care professional domain 320 includes an audiologist 322, a hearing care computer 324, and an pupillometric measurement device comprising goggles 326 for fittings at the clinic. The cloud service provider domain 330 includes data servers/centers 332.

Fig 6 illustrates an example of a (binaural) hearing system 700 according to the present disclosure comprising at least a left or/and a right hearing devices, a number of sensors mounted on a spectacle frame 710 of a pair of hearing aid glasses 318. The hearing aid system comprise a number of sensors S1i, S2i (*i*=1, ..., *N_{S}*) associated with (e.g. forming part of or connected to) the left and right hearing aid devices 310, 314. In one embodiment, as shown in fig 6, a first, second and third pair of sensors S₁₁, S₁₂, S₁₃ and S₂₁, S₂₂, S₂₃ are mounted on the spectacle frame 710 of the glasses. In the embodiment of FIG. 6, sensors S₁₁, S₁₂ and S₂₁, S₂₂ are mounted on the respective sidebars 711a, 711b, whereas sensors S₁₃ and S₂₃ are mounted on the cross bar 713 having hinged connections to the right and left side bars 711a, 711b. Lenses of the glasses 714 are mounted on the cross bar 713. The at least one hearing device 310, 314 comprises respective BTE (behind-the ear)-parts 712a, 712b. In one aspect the glasses comprise respective ITE (in-the-ear)-parts 712a, 712b. In one aspect, the ITE-parts comprise electrodes for picking up body signals from the user, e.g. forming part of sensors S1i, S2i (*i*=1, ..., *N_{S}*) for monitoring physiological functions of the user, e.g. brain activity or eye movement activity or temperature. The sensors mounted on the spectacle frame 710 may e.g. comprise one or more of an eye camera (e.g. for monitoring pupillometry), a blood sensor for measuring oxygen in the blood, a sensor for monitoring Temporomandibular joint (TMJ) movement and/or neck muscle (sternocleidomastoid) activity (e.g. a radar sensor).

The portable user device 316 communicatively interfaces with the cloud service 330 in order to provide data (such as measurement results, GPS data, questionnaires, etc.) from the user domain 310 to the cloud domain 330 and to receive data (audio data, video data, instructions, etc.) from the cloud domain 330. Hearing care computer 324 also interfaces with the cloud service 330 in order to upload data for the user 312 and download data from the user 312. Hence, the hearing aid user domain 310 is coupled to the hearing care professional domain 320 via cloud service 330.

In some examples, the proposed concept can comprise eHealth measured, user self-reported pre-fitting data as follows: self-reported user information on individualized expectations for hearing loss rehabilitation, individualized important listening situations, individualized preferences for sounds, etc. These data can be reported by the user 312 to the audiologist/cloud service through the cloud pre-fitting and/or at fine-tuning through the eHealth ecosystem 300 as shown in Fig. 3, for example using an app downloaded before fitting for getting reporting on important listening situations etc. The goggles or other eye tracker devices 318, 326 can be supplied by the clinic. Moreover, empowering rehabilitation information, learnings etc. could be sent from the audiologist/cloud to the users 312 through an app before fitting, thus preparing the users for the fitting. After fitting and in the important two to four weeks' acclimatization period, the cloud 330 could provide the audiologist 322 with objective user data on pupillometry measured by the goggles or other device 318 at the users' home, hearing aid usage, hearing aid satisfaction, rehabilitation problems and enable fast on-demand problem solving to the users.

Before doing the pupillometry measurement during the speech of samples, i.e. during the playing of the sound pattern in the hearing aid device 314 or in the headphone 314a, a baseline measurement has to be performed. The baseline measurement is a measure of the pupillary information, i.e. the pupil size and/or the pupil dilation, of the person under test without playing the sound pattern. The baseline measurement may be performed after each sound pattern.

Alternatively, the baseline measurement is a measure of the pupillary information, i.e. the pupil size and/or the pupil dilation, of the person in an environment which does not change. The environment is the space surrounding the person under test, for example the space may be within a room which the person is placed in during the actual measurement.

Alternatively, the baseline measurement is a measure of the pupillary information, i.e. the pupil size and/or the pupil dilation, of the person under test, where the user receives via the hearing aid device 314, 110 or the headphone 314a, 110a a background sound. The background sound, may be a white noise signal or any kind of a predefined background sound pattern. While playing the background sound a camera is measuring the pupillary information of the person, and the measure of the pupillary information is the baseline measurement.

The baseline measurement of the pupillary information, i.e. the pupil size and/or the pupil dilation, is used for normalizing the measured pupil dilation during the playing of the sound pattern including the background sound.

One example is described from the user's point of view step by step below:
1) Baseline measurement
   a. The user 312 can receive an app (frontend) with a connected cloud service (backend) with for example 25 listening effort sentences in e.g. four different hearing aid settings, background sound(s), reporting possibilities for important listening situations, etc.
   b. At home, the user 312 can receive the goggles 318 or alike and perform the baseline measurement by playing through the headphone worn by the user together with the goggles a background sound while measuring the pupil dilation of the person via the goggles 318. The baseline is then the measure of the pupil dilation when playing the background sound.
   c. Step b. shall be performed after each played sound pattern.
2) Fitting:
   a. The pupillometric goggles 318 can test the user through e.g. 25 listening effort sentences in e.g. four different hearing aid settings, while playing the background sound. The pupil dilation in these settings can be measured through the goggles or other device 318 with an eye tracker and post-processed. After each listening effort sentence a baseline measurement has to be performed. For each of the four settings, a mean pupil dilation as an indicator of the processing effort can be measured.
3) Follow-up:
   a. In an acclimatization period (e.g. two to four weeks) the user 312 can use the goggles or other device 318 to measure his/her pupillometric response as an indicator of his/her effort required for correct speech recognition with the fitted hearing aids. eHealth solutions can provide the user 312 with the listening tests through the cloud 330, as seen in Fig. 3.
   b. The hearing aid 314 can automatically be set using the test results of the different test, i.e. pupillometry, adjusting for their effort required for speech recognition in an aided listening situation. This automatic setting of the hearing aid 314 can take place in the clinic at the fitting session or at home for through settings sent by the cloud 330 through the eHealth ecosystem 300. Algorithms can set the hearing aids using the pupillometry etc. results. These data calculations and automatic adjustments can all be done through the eHealth ecosystem 300.
   c. To further individualize the hearing aid 314 to the user's real-life experienced important listening situations, a further pupillometric test using the user's own uploaded audio file (sent through the eHealth ecosystem, Fig. 3 from the user domain) could be used as a listening test and the hearing aid 314 could also automatically be set according to these tests.

Individualized hearing aid fitting through automated pupillometry could take place through listening tests send via a smartphone app and/or the hearing aids 314 and with measurements of pupil response through goggles or other device 318, 326. To improve measurement results the users 312 can be calibrated to the pupillometric tests at the fitting session in the clinic, before using the system at home in the two to four weeks' acclimatization period. So, first an individual baseline test can be performed since pupil dilation is usually very individual (see Fig. 4).

For some fitting scenarios, e.g. where we do not have predefined environmental conditions, it may be advantageous to know the light intensity at the eye. Then, the following acts can be performed when using pupillometry for hearing aid fitting:
- detecting the eye(s), e.g. via a conventional eye tracking scheme,
- measuring a first average light intensity at the eye(s),
- measuring a first pupil dilation,
- measuring a second average light intensity at the eye(s),
- measuring a second pupil dilation,
- correcting the second pupil dilation based on a difference between the first average light intensity and the second average light intensity.

Further, to solve the problem of mismatch between the clinical settings and real-life experiences, EMA (Ecological Momentary Assessment) data from the user's real-life experiences could be uploaded to the clinic/cloud through the eHealth ecosystem 300 as audio files and send as individualized listening tests through the hearing aids using pupillometry technology. Thereby, the users 312 can truly experience individualized fitting/fine-tuning from the users important listening situations.

The idea of using goggles 160, 318, 326 or the above adjusting for the light intensity mentioned above for the pupillometry tests can enable the technology to be used in non-controlled environments, as for instance different clinics, at home etc. and still giving the same measurement accuracy. Concerning the audio for the test through the hearing aids this can be controlled to have the same audio testing of all users. Thereby, we can eventually have population data to be used for population based clinical evidence refining the evidence for clinical decisions and for comparisons between age-groups etc.

Combining pupillometry and eHealth could deliver evidence based, individualized, and objective outcome measures data for the fitting process both in the clinics and at the user's homes. Pupillometry could be used as a clinical diagnostic tool for measuring hearing loss objectively. eHealth can support this clinical tool to be used also in the user's home in the multiple week's acclimatization period post-fitting. eHealth information and communication sharing between audiologists and their users is also used for individualization of the hearing loss rehabilitation process.

Instead of relying solely on subjective data, objective data coming from for instance pupillometry or hearing aids (usage, volume control etc.) could be used, lacking the biases which self-reported data are prone to, and thus providing data for evidence based clinical decision support.

While the aforementioned aspects address objective hearing aid fitting and provide solutions for objective remote hearing aid fitting, it is still very difficult to understand the daily world of people with hearing impairment and very difficult for people with hearing impairment to explain it to others. Significant others (for example, wife, husband, parent, child, friend, etc.) are the most important communication partners to most people with hearing impairment and the most important rehabilitation partner after hearing aid fitting. This places a high responsibility on the shoulders of the significant others in the hearing loss rehabilitation and daily life of their spouses with hearing loss. However, research has shown that they do not get the information or learning they need to live up to these challenges.

Furthermore, hearing loss is a very individual experience. Both with regards to individually important listening situations as well as perceived hearing loss. It is known that there are common important listening situations where most people with hearing impairment would like to be helped through their hearing loss and communication strategies, for instance a restaurant situation. But it is also known that today's hearing loss rehabilitation requires individualization. Therefore, it is important that communication tools can be individualized not only to the audiometric tests, but also to the individual's most important listening situations or current real-world communication situations, and here eHealth can be a solution.

Today's key actors in hearing rehabilitation - the audiologists, the significant others, and the people with hearing impairment do neither have access to common nor individualized data from virtual reality or real-world "laboratory" data regarding important listening situations to be used for communication training tools. Hence, the audiologists do not have any possibilities to fine-tune hearing aids or advice on communication problems and strategies from these kinds of experienced real world or virtual reality data.

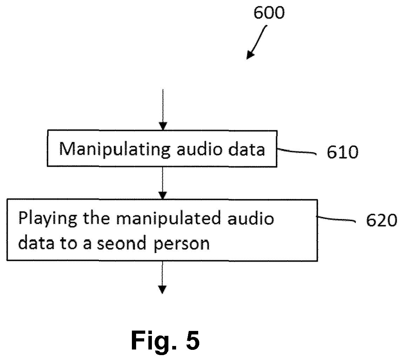

To overcome these and other problems, the present disclosure further provides a concept for emulating hearing loss, which can stand alone or be combined with the pupillometric fitting concept described above. Fig. 5 illustrates a flowchart of a corresponding hearing loss emulation method 600.

Method 600 comprises manipulating 610 audio data in accordance with audiometric data reflecting a first person's hearing loss, and playing back 620 the manipulated audio data to a second person (for example, the significant other). That is to say, all kinds of audio data can be manipulated in accordance with the audiometric data of the person with hearing loss in order to provide the hearing loss experience to others. For example, an audio file could be separated into different frequency ranges and each frequency range could be filtered/manipulated according to the audiometric data.

Thus, in some examples, manipulating 610 the audio data can comprise filtering the audio data with a filter having a transfer function reflecting the hearing loss. The filtering can be performed with a Digital Signal Processor (DSP), for example.

Some examples are illustrated in the following table showing the respective hearings loss of four persons for the left and right ear. It can be seen that person 2 has by far the strongest hearing loss over the whole frequency range.

| | **Left ear** | | | | | **Right ear** | | | | |
|---|---|---|---|---|---|---|---|---|---|---|
| **Frequency** | 250 | **500** | **1000** | **2000** | **4000** | **250** | **500** | **1000** | **2000** | **4000** |
| Person 1 hearing loss in dB | 35 | 35 | 40 | 55 | 65 | 50 | 45 | 45 | 65 | 70 |
| Person 2 hearing loss in dB | 65 | 70 | 75 | 70 | 70 | 80 | 90 | 90 | 65 | 70 |
| Person 3 hearing loss in dB ; | 10 | 25 | 40 | 50 | 60 | 15 | 30 | 40 | 50 | 55 |

In some examples, manipulating 610 the audio data can comprise manipulating predetermined audio data and storing the manipulated audio data for later playback. For example, the manipulated audio data can be stored in computer memory of a digital hardware device, such as a smartphone or a virtual reality headset.

Instead of storing predetermined audio data, manipulating 610 the audio data can alternatively include manipulating audio data of a current listening situation in real-time. In computer science, real-time computing describes hardware and software systems subject to a "real-time constraint", for example from event to system response. Real-time programs must guarantee response within specified time constraints, often referred to as "deadlines". That is to say, manipulating the audio data can take place within less than 500 ms, less than 200 ms, or even less than 50 ms, for example.

In some examples, manipulating 610 the audio data and playing back 620 the manipulated audio data can be performed using a noise-cancelling type headphone. Such an active headphone can be configured manipulate ambient sounds using active sound control. It can in-corporate one or more microphones (for directivity) and circuitry for generating audio data corresponding to environmental sound. A digital signal processor of the headphone can be configured to manipulate/filter the audio data in accordance with a transfer function reflecting the hearing loss. In some examples, the audio data is manipulated in accordance with directional data and/or distance data corresponding to current movement and/or head orientation and/or distance of the second person. Thus, a dB output can be decreased according to directional data and/or distance data, for example. Real-world situations can thus be presented to the significant other through a multi microphone headset, which could also be worn outside the home for instance in restaurants for the significant other to experience this often very difficult listening situation from the hearing impaired spouse's perspective.

In some examples, the manipulated audio data can be played back to the second person via a virtual reality headset. In some examples, playing back 620 the manipulated audio data can further comprise displaying video data along with and/or corresponding to the audio data. Thus, a virtual reality scenario can be created for the second person. The common or the individually important listening situations with the manipulated sounds to reflect their spouses hearing loss can thus be shown to the significant other through the virtual reality headset.

In some examples, the method 600 can further comprise transmitting audio-visual data corresponding to a real-life experience of the first person to a remote server, and manipulating, at the remote server, an audio portion of the audio-visual data in accordance with the audiometric data of the first person. For example, a person with hearing impairment can upload videos to the cloud/audiologist of individually important listening situations using an app or just a smartphone.

In some examples, the method 600 can further include transmitting the manipulated audio-visual data from the remote server to a mobile display device of the second person. The mobile display device can be a smartphone, a computer, a virtual reality headset, or the like.

According to a further aspect, the present disclosure also provides a computer program for performing the method 600 for emulating hearing loss, when the computer program is executed on a programmable hardware device.

According to a further aspect, the present disclosure also provides an apparatus for emulating hearing loss. The apparatus comprises a processor which configured to manipulate audio data in accordance with audiometric data of a first person suffering from hearing loss, and a play back device configured to play back the manipulated audio data to a second person. The skilled person having benefit from the present disclosure will appreciate that the apparatus can be comprised of a computer, a smartphone, a virtual reality headset, a multi micro-phone headset, or the like.

A more detailed example of the proposed concept can come in three steps:
1) First, a virtual reality headset can be used with capabilities to replicate individualized hearing impairment by sound manipulation of selected hearing conditions, e.g., common important listening situations and reflecting manipulated sounds when persons/things are located differently to the sound source.
   The sound manipulation can be done to reflect the hearing impaired user's 312 hearing loss in accordance with the audiometric tests done when the hearing impaired user 312 was fitted with hearing aids (the audiogram results), e.g., the sounds is de-graded to reflect the hearing loss (for instance lowered 40 dB for frequencies of 2000 Hz, 60 dB for frequencies of 4000 Hz, etc. and with specific loudness).
   Moreover, the sounds can also be degraded in accordance to the movements/location experienced when watching the videos with important listening situations, e.g., degrading sounds when someone speaks with their back turned to a person with hearing impairment or speaks from another room, etc. This can train communication strategies for the person with hearing impairment and especially the significant other in the most common important listening situations and serve as an evidence based decision support tool for audiologists, as all clients and significant others can use the same common listening situations in this first step of communication strategies training.
2) Secondly, we can go for individualization of the training in communication strategies. The person with hearing impairment can upload videos to the cloud/audiologist of individually important listening situations using an app or just a smartphone (see the hearing aid user domain in Fig. 3).
   These individually important listening situations can be used for individualized communication training strategies through a virtual reality headset, for example. For this purpose, the cloud/audiologist can upload the videos to the virtual reality system and these videos can be sound manipulated to reflect the hearing impaired user's 312 hearing loss in accordance with the audiometric tests done when the user 312 was fitted with hearing aids (the audiogram results).
3) In this third step, the hearing impaired user 312 and the significant other have trained both common and individualized important listening situations in step 1 and 2 and have experienced -through virtual-reality- what happens to the sounds and your communication situations when you are positioned differently to the sounds you want to listen to. But the training has so far been through virtual-reality and now it is time to move out in the real world and learn more.
   This can be done via noise cancelling type headsets, so external sound can be suppressed, and with at least two microphones on each earpiece for directionality purposes and at least one receiver in the earpieces. Once we have suppressed the external sound, the system can amplify what is heard from the mics in each ear, but does so reflecting the hearing impaired spouse's hearing loss in accordance with the audiometric tests done when the spouse was fitted with hearing aids (the audiogram results).
   Now the significant other can engage in real-world communication situations with their hearing impaired spouse or in other situations experiencing their spouses hearing loss and learning from own experiences how to communicate with people with hearing loss.

In some examples, the virtual reality headset and the multi microphone headset can both operate in a cloud based eHealth ecosystem 300 allowing the integration of the three functional domains: 1) Hearing Aid User Domain 310; 2) Audiologist/Healthcare Professional Domain 320; and 3) Cloud Service Provided Domain 330. The virtual reality headset can get videos on common and individually important listening situations from the cloud service provider domain 330. The common important listening situation videos could for instance be uploaded from a video library. In the cloud 330 all types of videos can be sound manipulated in accordance with the audiometric tests of the individual hearing impaired user 312. The individually important listening situations can be uploaded from the hearing impaired user 312 to the cloud/audiologist via a smartphone 316, for example. In one aspect, the sound manipulation of the multi-microphone headset can be done through the cloud 330 and/or the significant others smartphone.

The significant other can use the virtual reality headset at home. The common or the individually important listening situations with the manipulated sounds to reflect their spouses hearing loss can be shown to the significant other via the virtual reality headset. The real-world situations are presented to the significant other via a multi microphone headset, which could also be worn outside the home for instance in restaurants for the significant other to experience this often very difficult listening situation from the hearing impaired spouse's perspective. The tools can also solve the problems of people with hearing loss having very large difficulties in explaining their hearing loss and its implications in communicating with the world. The audiologist can with these tools have a pool of virtual-world "laboratory" tests and thereby a pool of scenarios for their clinical decision support and advisory service on communication strategies, enabling evidence based and uniform clinical service and sup-port to people with hearing loss and their significant others. Furthermore, the audiologist can have access to individually important listening situations for refining of the communication strategies training and understanding and insight in the individual problems of clients.

The solution brings both the person with hearing impairment and the significant other in play in the individualized rehabilitation process and empowers the significant other as an important partner in the rehabilitation process.

The aspects and features mentioned and described together with one or more of the previously detailed examples and figures, may as well be combined with one or more of the other examples in order to replace a like feature of the other example or in order to additionally introduce the feature to the other example.

Examples may further be or relate to a computer program having a program code for performing one or more of the above methods, when the computer program is executed on a computer or processor. Steps, operations or processes of various above-described methods may be performed by programmed computers or processors. Examples may also cover program storage devices such as digital data storage media, which are machine, processor or computer readable and encode machine-executable, processor-executable or computer-executable programs of instructions. The instructions perform or cause performing some or all of the acts of the above-described methods. The program storage devices may comprise or be, for instance, digital memories, magnetic storage media such as magnetic disks and magnetic tapes, hard drives, or optically readable digital data storage media. Further examples may also cover computers, processors or control units programmed to perform the acts of the above-described methods or (field) programmable logic arrays ((F)PLAs) or (field) programmable gate arrays ((F)PGAs), programmed to perform the acts of the above-described methods.

The description and drawings merely illustrate the principles of the disclosure. Furthermore, all examples recited herein are principally intended expressly to be only for pedagogical purposes to aid the reader in understanding the principles of the disclosure and the concepts contributed by the inventor(s) to furthering the art. All statements herein reciting principles, aspects, and examples of the disclosure, as well as specific examples thereof, are intended to encompass equivalents thereof.

A functional block denoted as "means for ..." performing a certain function may refer to a circuit that is configured to perform a certain function. Hence, a "means for s.th." may be implemented as a "means configured to or suited for s.th.", such as a device or a circuit configured to or suited for the respective task.

Functions of various elements shown in the figures, including any functional blocks labeled as "means", "means for providing a sensor signal", "means for generating a transmit signal.", etc., may be implemented in the form of dedicated hardware, such as "a signal provider", "a signal processing unit", "a processor", "a controller", etc. as well as hardware capable of executing software in association with appropriate software. When provided by a processor, the functions may be provided by a single dedicated processor, by a single shared processor, or by a plurality of individual processors, some of which or all of which may be shared. However, the term "processor" or "controller" is by far not limited to hardware exclusively capable of executing software, but may include digital signal processor (DSP) hardware, network processor, application specific integrated circuit (ASIC), field programmable gate array (FPGA), read only memory (ROM) for storing software, random access memory (RAM), and non-volatile storage. Other hardware, conventional and/or custom, may also be included.

A block diagram may, for instance, illustrate a high-level circuit diagram implementing the principles of the disclosure. Similarly, a flow chart, a flow diagram, a state transition diagram, a pseudo code, and the like may represent various processes, operations or steps, which may, for instance, be substantially represented in computer readable medium and so executed by a computer or processor, whether or not such computer or processor is explicitly shown. Methods disclosed in the specification or in the claims may be implemented by a device having means for performing each of the respective acts of these methods.

It is to be understood that the disclosure of multiple acts, processes, operations, steps or functions disclosed in the specification or claims may not be construed as to be within the specific order, unless explicitly or implicitly stated otherwise, for instance for technical reasons. Therefore, the disclosure of multiple acts or functions will not limit these to a particular order unless such acts or functions are not interchangeable for technical reasons. Further-more, in some examples a single act, function, process, operation or step may include or may be broken into multiple sub-acts, -functions, -processes, -operations or-steps, respectively. Such sub acts may be included and part of the disclosure of this single act unless explicitly excluded.

Furthermore, the following claims are hereby incorporated into the detailed description, where each claim may stand on its own as a separate example. While each claim may stand on its own as a separate example, it is to be noted that - although a dependent claim may refer in the claims to a specific combination with one or more other claims - other examples may also include a combination of the dependent claim with the subject matter of each other dependent or independent claim. Such combinations are explicitly proposed herein unless it is stated that a specific combination is not intended. Furthermore, it is intended to include also features of a claim to any other independent claim even if this claim is not directly made dependent to the independent claim.

## Claims

1. Method for adjusting a hearing aid device, the method comprising:
• providing a sound pattern to a person wearing the hearing aid device configured according to current hearing aid settings;
• evaluating the person's pupils to obtain pupillary information in response to the sound pattern perceived via the hearing aid device; and
• adjusting the current hearing aid settings based on the pupillary information.

2. The method of claim 1, wherein providing the predefined sound pattern comprises playing predefined speech samples to the person wearing the hearing aid device.

3. The method of any of the previous claims, wherein before providing the predefined sound pattern a background sound is provided to the person, wherein a baseline measurement of the pupil is provided in response to the background sound, and wherein the evaluating of the person's pupils to obtain pupillary information is obtained in response to the sound pattern mixed with the background sound.

4. The method of any one of the previous claims, wherein providing the sound pattern comprises transmitting data indicative of the sound pattern from a remote device to a mobile device associated with the person wearing the hearing aid device.

5. The method according to claim 3, wherein the background sound comprises a predefined background sound, such as a white noise sound.

6. The method of any one of the previous claims, wherein evaluating the person's pupils comprises measuring a pupil dilation.

7. The method of claim 2, wherein evaluating the person's pupils comprises normalizing the pupil dilation using the measured pupil in response to the background sound, where the measured pupil comprises the pupil dilation.

8. The method of any one of the previous claims, wherein the person's pupils are evaluated using an eye tracking device or pupilometer and/or a post process involving eye-blink removable, artifact removable, data interpolation and/or a normalizing of the pupil dilation using the measured pupil in response to the background sound, where the measured pupil comprises the pupil dilation.

9. The method of claim 8, wherein the eye tracking device or pupilometer comprises pupillometric goggles or a mobile device including a photodetector and a signal processor with an eye tracking software.

10. The method of any one of the previous claims, wherein adjusting the current hearing aid settings comprises varying the current hearing aid settings to decrease a measured pupil dilation in response to the sound pattern.

11. The method of any one of the previous claims, wherein adjusting the current hearing aid settings comprises choosing the current hearing aid settings from a plurality of different predetermined hearing aid settings and selecting the hearing aid settings causing the least pupil dilation in response to the sound pattern.

12. The method of any one of the previous claims, further comprising
• transmitting the pupillary information to a processor; and
• providing adjusted hearing aid settings by the processor based on the pupillary information.

13. A computer program for performing the method of any one of the previous claims, when the computer program is executed on a programmable hardware device.

14. Apparatus for adjusting a hearing aid device, the apparatus comprising:
• a receiver configured to receive pupillary information from a person wearing the hearing aid device configured according to current hearing aid settings; and
• a processor configured to generate adjusted hearing aid settings based on the pupillary information.

15. System for adjusting a hearing aid device, the system comprising:
• means for providing a sound pattern to a person wearing the hearing aid device configured according to current hearing aid settings;
• means for evaluating the person's pupils to obtain pupillary information in response to the sound pattern perceived via the hearing aid device; and
• means for adjusting the current hearing aid settings based on the pupillary information.

16. The system for adjusting a hearing aid device according to claim 15, wherein the means for providing a sound pattern to a person wearing the hearing aid device form part of or are mechanically and/or electrically connected to the means for evaluating the person's pupils.

17. The system for adjusting a hearing aid device according to claim 15, wherein the means for providing a sound pattern to a person wearing the hearing aid device consists of or comprise hearing aid, headset, earphone or a combination thereof.

18. The system for adjusting a hearing aid device according to claim 15, wherein the means for evaluating the person's pupils consists of or comprise glasses or goggles.
